# EUROPEAN PATENT APPLICATION

(11) **EP 1 913 923 A1**
(43) Date of publication of application: **23.04.2008**
(21) Application number: 07020601.6
(22) Date of filing: 19.10.2007
(51) Int. Cl.: A61H 31/00

(54) **Resuscitation system**

(30) Priority: 20.10.2006 US 584257; 20.10.2006 NO 20064768
(71) Applicant: Laerdal Medical AS, 4002 Stavanger (NO)
(72) Inventor: Myklebust, Helge, 4025 Stavanger (NO); Stromsnes, Oystein, 4120 Tau (NO)
(74) Representative: HOFFMANN EITLE

(57) **Abstract**

The invention regards a resuscitation system comprising a chest compression device to repeatedly compress the chest of a patient and thereafter causing or allowing the chest to expand, a defibrillator to apply electric impulses to the heart, measuring devices for measuring characteristics of the resuscitation process, and a signal processor for controlling operation of the chest compression device and/or the defibrillator. The defibrillator may be an integrated or external device working in a master/slave relationship with the remaining system.

## Description

This invention is related to resuscitation systems.

Sudden cardiac arrest is a leading cause of death in developed countries in the Western World, like United States and Canada.

To increase the chance for survival from cardiac arrest, important aspects are CPR (Cardio Pulmonary Resuscitation) and heart defibrillation given in the first few critical minutes after the incident.

CPR is performed to ensure a sufficient flow of oxygenated blood to vital organs by external compression of the chest combined with rescue breathing. Heart defibrillation is performed to re-establish normal heart rhythm by delivery of an external electric shock.

The *quality* of CPR is essential for survival. Chest compressions must be given with a minimum of interruptions, and be of sufficient depth and rate. Performing chest compressions manually is an extremely exhausting task, and it is practically impossible to give manual CPR of sufficient quality during transportation of a patient.

A successful defibrillation depends on the metabolic state of the heart when the electrical shock is applied. It must be performed at a determined times during the compression cycle. In general defibrillators comprise an integrated (or external) electrocardiograph device (ECG) which records the electrical activity in the heart in a continuous manner and permits determination of the correct time for applying an electrical shock. Chest compressions cause noise in the ECG signal and can lead to incorrect determination of the time for defibrillation. For this reason, in most cases, chest compressions are performed on a patient either manually or automatically and the process is stopped in order to get a correct ECG signal before applying defibrillation. This leads to relatively long periods without chest compression and to cumbersome procedures where different equipment must placed on the patient.

US 2006/0155222 describes a device for performing chest compressions for CPR in coordination with applying electrostimulus for additional resuscitative actions such as defibrillation. In this publication defibrillation is applied near the end of a compression cycle. This publication describes only timing of the defibrillator shock with respect to the CPR cycle.

US6807442 describes a system for reducing signal disturbances in ECG, which disturbances are caused by cardio-pulmonary resuscitation (CPR). The system includes a measuring device for measuring one or more signals derived from parameters such as compression depth, lung inflation etc. as a result of CPR. The parameter signals form one or more reference signals that correlate with the signal disturbances. One or more adaptive filters filter the signals out from the signal that constitutes the ECG signals so as to remove disturbances caused by CPR.

Said publications do not address the problem of providing a resuscitation system where signals representing characteristics of the resuscitation process are adapted for use in controlling the process.

The object of the invention is to provide a resuscitation system which solves the above problem and which minimizes hands-off-time during a resuscitation procedure.

This problem is solved by means of a system according to the invention, comprising a chest compression device to repeatedly compress the chest of a patient and thereafter causing or allowing the chest to expand, a defibrillator to apply electric impulses to the heart, measuring devices for measuring characteristics of the resuscitation process, and a signal processor for controlling operation of the chest compression device and/or the defibrillator.

The resuscitation system can comprise an integrated defibrillator or a docking station and/or connection(s) for receiving and/or interfacing an external defibrillator. The alternative using an external defibrillator will permit use of a system according to the invention together with any defibrillators available in the market. The connection between the defibrillator and the remaining system may be wired or wireless. The defibrillator may have its own power supply or may share power supply with the system.

The processor device can be adapted to control operation of the chest compression device and/or the defibrillator based on measured characteristics of the resuscitation process. The processor device can also be adapted to control operation of the chest compression device and/or the defibrillator based on predetermined characteristics of the resuscitation process, or it can be adapted to control operation of the chest compression device and/or the defibrillator based on comparison of measured and predetermined characteristics of the resuscitation process. The predetermined characteristics may for example be characteristics recommended in the international guidelines for resuscitation.

The present international guidelines describes the recommended time for activating defibrillation during CPR. After having performed the recommended period of time of CPR, for example three minutes, the defibrillation is activated, the result of the defibrillation is analyzed, and CPR is continued for another period of time if the heart still has no rhythm. It is desirable to minimize the time intervals between CPR and defibrillation and between defibrillation and continued CPR, and this may be achieved by a common processor for the CPR devices and defibrillator. For example may the resuscitation system according to the invention allow continuous CPR and activation of defibrillator during CPR. Other possible predetermined characteristics are a recommended number of compressions before defibrillation, presumed state of heart according to time from stop, characteristics of ECG such as "slope", presence of VF, etc.

The processor device may be integrated in the system or may be a part of an external defibrillator. When connected together, the control operations may be performed by the defibrillator, the defibrillator being the "master" and the remaining system a "slave", or the control operations may be performed by the system, the system being "master" and the defibrillator "slave".

The measuring device may be any sensors or other measuring devices suitable for measuring characteristics of the resuscitation process, and other relevant information regarding CPR in the system and/or in the patient. Such sensors/measuring devices are for example force sensors and/or depth sensors for measuring force/depth exerted/traveled by the compression device, compression counters, compression frequency counters, blood flow sensors for monitoring the blood flow of the patient, ventilation sensors for monitoring the ventilation flow, volume, and/or time interval of patient ventilation, impedance measuring means for measuring the impedance of the chest and thus give an indication of the ventilation of the patient, electrocardiogram (ECG) device, tilt sensors for measuring the angle of the patient (whether the patient is lying, sitting/standing), position detectors for detecting the positioning and/or change of positioning of the chest compression means, battery power measurement means, internal motor temperature measuring means, etc.

The resuscitation system according to the invention may also comprise ventilation devices. The ventilation devices may be regular ventilation devices which may be operated by an operator, or they may be autonomous/automatic ventilation devices. In the case where the ventilation devices are operated by an operator, the resuscitation system may comprise a sensor for measuring characteristics (quality) of the ventilation, such as ventilation rate and volume.

The defibrillator in the system according to the invention can comprise an ECG device or said device can be a part of the measuring devices.

The system can comprise power supply devices comprising energy storage devices or devices for connection to power sources in an ambulance, in a hospital or in an external power storage device.

The system according to the invention can further comprise data storing devices connected to the measuring devices and/or the defibrillator for storing values of the characteristics measured by the measuring device and/or an ECG device in the defibrillator. These stored values may later be used for evaluating the resuscitation episode in order to be able to adjust procedures and/or train personnel. Stored values may also be used to reveal equipment errors and/or initiate service.

The resuscitation system according to the invention may further comprise a user interface for providing information regarding the resuscitation to the helping personnel. The user interface may be comprised in or be separate from the feedback devices. The user interface may provide information related to the service level of the system, remaining power, defibrillator status, ventilation status or other information which would be useful for the operator during or after the resuscitation.

The defibrillator device in the system according to the invention can be an automatic external defibrillator (AED).

The system according to the invention can comprise a support connected to the chest compression device.

The chest compression device may be any device suitable for compressing the chest of a patient, such as pneumatic, hydraulic, or electric actuated pistons, belts, straps, etc. The chest compression device may be fixed to the patient's chest/skin by means of fastening devices such as tape or by vacuum, or it can be merely in contact with the chest without being fastened to the chest. The chest compression device can be designed to cause the chest to expand, i.e. is to perform an active lifting of the chest, or to allow the chest to expand freely.

Besides, the chest compression device comprises or is connected to a support in order to maintain a substantially constant positioning of the chest compression device on the patient's chest. A substantially constant positioning of the chest compression device on the patient's chest is important in order to obtain the necessary quality of the compressions and for safety reasons.

Said support may be adapted to be arranged under the patient, on one of the sides, or reaching round the patient.

In one embodiment, the support comprises two legs connected to a back plate and to a transverse plate. The back plate is adapted for placement under the patient when the patient is lying, and the two legs are adapted for placement on both sides of the patient. The width and the height of the quadrangle may be adjusted to fit each patient.

The support should be symmetric and surround the patient closely in order to give a stable support and ensure correct positioning of the compression device and thus the compressions on the patient.

In one embodiment of the invention the compression device is firmly and fixed connected to the transverse plate in a position which ensures correct positioning on the patient's chest. In an alternative embodiment, the compression device may constitute the transverse plate completely or partially. The compression device is connected to the support in such a way that the direction of the compression movement of the chest compression device is substantially perpendicular to thorax in the area between the nipples. This may for example mean that the movement of the compression device is substantially perpendicular to a plane comprising sternum, and substantially parallel to the back plate.

The positioning of the patient relative to the support is partly or fully a inherent property of the support. The positioning relative to the patient's width may be performed by arranging the legs of the support close in contact with the patient's sides in the embodiments of the support comprising legs. The positioning relative to the patient's length may be performed by means of illustrations on the support or by other display devices and supervised by the operator. In one embodiment the system may comprise physical devices or arrangements which indicates and/or guides the positioning of the system relative to the patient, for example by arranging the legs in the patient's armpits, by a rod indicating the distance to the patient's shoulders, etc.

In other embodiments, the support can be a frame, stand, rack, tripod, etc. of suitable design.

The support may be collapsible, demountable or foldable in order to minimize volume of the system when not in use. Preferably, the support is easy to assemble and prepare for use in order to minimize time wasted on assembling and mounting. This may for example be achieved by using spring-loaded elements which unfold themselves to the maximum size. The system should comprise as few separate parts as possible in order to minimize risk of incorrect assembly and to minimize assembly time. In one embodiment the system only comprises two separate sections for assembly.

Although different features of the invention are described in the present specification as belonging to different embodiments it will be clear for the skilled person that all or some of these can be combined into a single embodiment.

The invention will now be described in more detail by devices of examples and with reference to the attached figures, where:
Figure 1 is a block diagram of an embodiment of the invention,
Figure 2 is a perspective view of an embodiment of the invention.
Figure 3 shows an example of VF signals and signals caused by CPR.
Figure 4 illustrates an exemplary decision model.

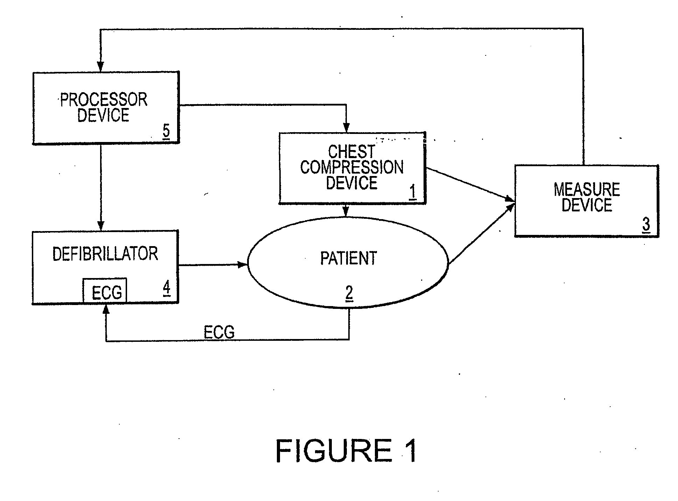

Figure 1 is a block diagram of an embodiment of the resuscitation system according to the invention. This block diagram shows chest compression devices 1 for repeatedly compressing the chest of a patient 2 and causing or allowing the chest to expand, measuring devices 3 for measuring characteristics of the resuscitation process, a defibrillator with an ECG device 4 and a signal processor 5 connected to the measuring devices 3 and/or the chest compression devices 1 and/or the defibrillator 4, to control operation of the chest compression devices 1.

In this embodiment of the invention the signal processor 4 is connected to data storing devices 5 to permit storage of measurement values and thus provide historical data, and to display devices 6 to display characteristics of the resuscitation process, and/or alarms.

The processor device in this embodiment of the invention is adapted to receive signals from the measuring device 3 and the ECG in defibrillator 4, to filtrate chest compression noise and based on the filtered signal to provide either an indication to a user of the correct time to defibrillate or a start signal to the defibrillator.

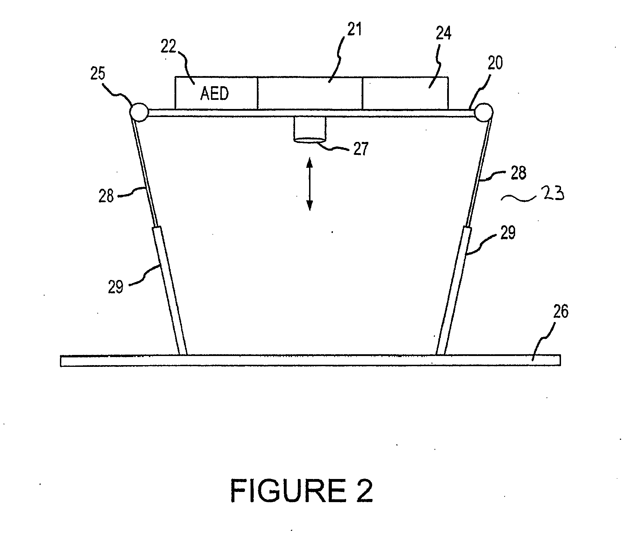

Figure 2 is a perspective view of an embodiment of the invention. In this embodiment of the invention, the chest compression device 21, the signal processor, the power supply and the defibrillator 22 are situated on a transverse plate 20. The signal processor and the power supply are enclosed in a housing 24. The defibrillator 22 is in this illustration an automated external defibrillator (AED) which is integrated with the other elements of the system according to the invention. The AED 22 communicates seamless with the other elements/components of the system.

The chest compression devices comprise in this embodiment of the invention a piston 27, a transmission mechanism for transmitting energy to the piston 27 and a motor (not shown). Piston 27 moves in the direction of the arrow and is in charge of performing compression and allowing or performing decompression of the patient's chest.

The signal processor comprises in this embodiment of the invention devices for controlling operation of the chest compression devices and the AED based on predetermined characteristics and/or on characteristics measured by measuring devices (not shown). These characteristics can be, as mentioned before force exerted by the piston, depth traveled by the piston, compression frequency, blood flow in the patient, ventilation flow, etc.

The above mentioned devices are situated on a transverse plate 20. Transverse plate 20 is substantially rectangular and is connected on its short edges to two lateral legs 23. The connection between transverse plate 20 and the legs 23 is implemented by hinges 25, this permitting to rotate the legs towards the transverse plate to provide a storage position for the resuscitation system. The legs comprise an upper part 28 and a lower part 29. The upper part 28 is hinged to the transverse plate 20 and is also situated telescopically inside the lower part 29. This arrangement permits easy step less variation of the legs' length. The legs are adapted for placement on the sides of the patient's body. The lower edge of the lower part 29 is connected to a back plate 26 adapted for placement under the patient's back.

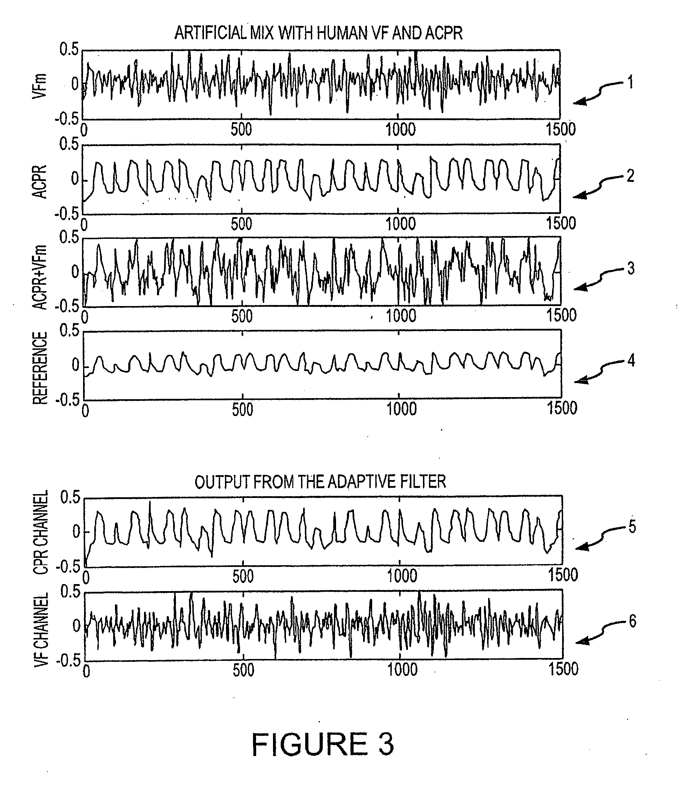

Figure 3 shows an example of ventricular fibrillation (VF) signals and signals caused by CPR. Curve 1 shows a VF curve from a person without signal disturbance. Curve 2 shows signal disturbances during CPR measured on a pig. Curve 3 shows the sum of signals 1 and 2 and simulates/represents a VF signal disturbed by CPR. Curve 4 shows a reference signal which is provided by position and impedance measurements. These measurements may be used to estimate the disturbance signal from CPR, an example of such an estimate is shown as curve 5. Subtracting the estimated disturbance from the disturbed VF signal (i.e. curve 3), provides an estimated "undisturbed" curve 6 which may be used for deciding when to activate the defibrillator.

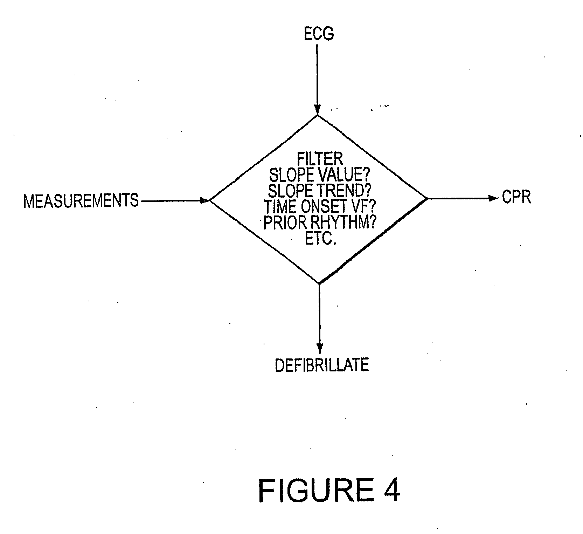

An exemplary decision model is shown in figure 4. The signals from ECG and CPR sensors are used to provide an estimated ECG curve, which is analysed with respect to for example slope characteristics, VF detection, prior rhythm, etc. The slope value can give an indication of the effect of defibrillation, a high value indicates good effect, while a low value indicates poor effect. A positive trend of the slope may indicate that ongoing CPR should be continued. The estimated ECG curve may be provided continuously or intermittent. The analysis of the estimated ECG curve leads to a decision on whether CPR should be performed or a defibrillator shock should be activated.

## Claims

1. Resuscitation system, comprising:
- a chest compression device to repeatedly compress the chest of a patient and thereafter causing or allowing the chest to expand, and
- a defibrillator to apply electric impulses to the heart,
- measuring devices arranged for measuring characteristics of the resuscitation process, and
- a signal processor arranged for controlling operation of the chest compression device and/or the defibrillator.

2. Resuscitation system according to claim 1,
**characterised in that** the processor device is adapted to control operation of the chest compression device and/or the defibrillator based on measured characteristics of the resuscitation process.

3. Resuscitation system according to claim 1,
**characterised in that** the processor device is adapted to control operation of the chest compression device and/or the defibrillator based on predetermined characteristics of the resuscitation process.

4. Resuscitation system according to claim 1,
**characterised in that** the processor device is adapted to control operation of the chest compression device and/or the defibrillator based on comparison of measured and predetermined characteristics of the resuscitation process.

5. Resuscitation process according to claim 1,
**characterised in that** the processor device comprises a filter for attenuating signal noise caused by chest compression in an ECG and/or impedance signal.

6. Resuscitation system according to claim 1,
**characterised in that** the measuring devices comprise a force sensor and/or a depth sensor.

7. Resuscitation system according to claim 1,
**characterised in that** the measuring devices comprises a blood flow and/or blood pressure sensor.

8. Resuscitation system according to claim 2, where the measuring devices comprises a ventilation sensor.

9. Resuscitation system according to claim 1, comprising ventilation devices.

10. Resuscitation system according to claim 1,
**characterised in that** the measuring devices or the defibrillator comprises an ECG device.

11. Resuscitation system according to claim 1,
**characterised in that** the power supply devices comprise energy storage devices or devices for connection to power sources in an ambulance, in a hospital or in an external power storage device.

12. Resuscitation system according to claim 1,
**characterised in in that** it further comprises data storing devices connected to the measuring devices for storing values of the characteristics measured by the measuring device.

13. Resuscitation system according to claim 1,
**characterised in that** it further comprises a user interface for providing information regarding the resuscitation to the operator.

14. Resuscitation system according to claim 1,
**characterised in that** the defibrillator device is an automated external defibrillator (AED).

15. Resuscitation system according to claim 1,
**characterised in that** the system further comprising a support connected to the chest compression device.
